# EUROPÄISCHE PATENTSCHRIFT

(11) **EP 0 976 372 B1**
(45) Veröffentlichungstag und Bekanntmachung des Hinweises auf die Patenterteilung: **15.10.2003**
(21) Anmeldenummer: 99113809.0
(22) Anmeldetag: 15.07.1999
(51) Int. Cl.: A61F 11/08

(54) **Gehörschutz mit einem im Ohr einsetzbaren Gehäuse**
Internal protective device for the ear
Dispositif interne de protection pour les oreilles

(30) Priorität: 29.07.1998 DE 19834146
(43) Veröffentlichungstag der Anmeldung: 02.02.2000
(73) Patentinhaber: Schinko-Neuroth Ges.m.b.H., 8421 Wolfsberg (AT)
(72) Erfinder: Hammerschmied, Rudolf, 5760 Saalfelden (AT)
(74) Vertreter: Cohausz & Florack

(56) Entgegenhaltungen:
- DE-A- 1 925 495
- DE-C- 251 598
- DE-C- 298 293
- DE-U- 9 313 061
- DE-U- 29 808 467
- FR-A- 1 188 716
- US-A- 2 327 620
- US-A- 3 800 101

## Beschreibung

Die Erfindung betrifft einen Gehörschutz aus einem dem Ohreingang angepaßten Gehäuse und einem darin untergebrachten Ventil, das zwischen mindestens einem von außen ausgehenden Belüftungs- und Schallkanal und einem zur Innenseite führenden zentralen Kanal angeordnet ist, dessen Eingang als Ventilsitz für dessen von außen über eine Stellschraube einstellbaren Ventilkörper ausgebildet ist. Ein Gehörschutz gemäß dem Oberbegriff des Anspruchs 1 ist aus DE-A-298 293 bekannt.

Um Personen vor Lärm zu schützen, sind verschiedene Arten von Gehörschutz bekannt. Entweder werden die Ohrmuscheln vollständig eingekapselt oder Stöpsel werden in die Ohreingänge eingesetzt. Die zweite Art von Gehörschutz ist für den Träger angenehmer als die erste Art, weil sie weniger voluminös ist und in Verbindung mit einem Paßstück individuell dem Gehöreingang des Trägers angepaßt werden kann. Zu diesem der zweiten Art angehörenden Gehörschutz, der auch unter "Otoplastik" bekannt ist, gehört vor allem ein Gehörschutz der eingangs genannten Art, der insofern vorteilhaft ist, weil er die Möglichkeit bietet, den Grad der Luft- und Schalldurchlässigkeit einzustellen.

Bei einem bekannten Gehörschutz der eingangs genannten Art (US-PS 3,702,123) sind der Ventilsitz und der Ventilkörper konisch ausgebildet. Auf verhältnismäßig kurzem Stellweg lassen sich große Unterschiede im freien Querschnitt einstellen. Eine feinfühlige Einstellungerlaubt diese Ausbildung des Ventils deshalb nicht. Hinzu kommt, daß die Herstellung eines solchen Gehörschutzes aufwendig ist, denn für die Stellschraube ist im Gehäuse ein Gewindeeinsatz vorgesehen, in dem mindestens der eine von außen ausgehende Belüftungs- und Schallkanal ausgebildet ist.

Bei einem anderen bekannten sehr ähnlichen Gehörschutz (DE-GM 93 13 061) sitzt die Stellschraube des Ventilkörpers mit ihrem Gewinde unmittelbar in einem entsprechenden Gewinde des Gehäuses. Der mindestens eine von außen ausgehende Belüftungs- und Schallkanal ist in der Stellschraube selbst ausgebildet. Bei diesem Gehörschutz ist der gesamte zur Innenseite führende zentrale Kanal konisch, während der Ventilkörper ebenfalls konisch ausgebildet ist aber eine geringere Konizität hat. In diesem Fall liegt der Ventilsitz am Ausgang des zentralen Kanals. Da das Gewinde aus herstellungstechnischen Gründen schon ein gewisses Spiel hat, wird der Ventilkörper nicht exakt zentral geführt. Unter dem Schalldruck kann es zum Flattern des Ventilkörpers kommen, was mit einer unangenehmen Geräuschentwicklung für den Träger verbunden ist. Die fehlende Führung und der nur lockere Sitz durch das Gewinde haben auch negative Auswirkungen auf die Feinfühligkeit der Einstellung und können dazu führen, daß sich der Ventilkörper löst und sogar ganz verlorengeht.

Der Erfindung liegt die Aufgabe zugrunde, einen Gehörschutz der eingangs genannten Art zu schaffen, der sich mit hoher Feinfühligkeit einstellen läßt, ohne daß dafür ein großer vorrichtungstechnischer Aufwand erforderlich ist.

Diese Aufgabe wird erfindungsgemäß dadurch gelöst, daß der Ventilsitz als zylindrische Bohrung und der Ventilkörper als ein darin geführter zylindrischer Zapfen ausgebildet sind, wobei zwischen der Bohrung und dem Zapfen mindestens eine sich in axialer Richtung erstreckende und in Richtung zur Innenseite im Querschnitt zunehmende Nut vorgesehen ist, deren freier Durchgangsquerschnitt von der Axialstellung des Zapfens zu der Bohrung abhängt.

Der erfindungsgemäße Gehörschutz ist einfach herzustellen, weil im Gehäuse nur zylindrische Bohrungen und Körper vorgesehen sind. Auch die Nut, die als Außennut vorzugsweise im Zapfen ausgebildet ist, läßt sich problemlos einformen. Ihr in Achsrichtung unterschiedlicher Querschnitt kann durch unterschiedliche Tiefe und/oder Breite erhalten werden. Auch ist eine feinfühlige Einstellung gewährleistet, weil der Zapfen in der zylindrischen Bohrung geführt wird. Eventuelles Spiel im Gewinde der Stellschraube kann sich dann nicht mehr so stark wie bei dem bekannten Gehörschutz auswirken.

Nach einer Ausgestaltung der Erfindung ist zwischen dem Ausgang des Belüftungs- und Schallkanals und dem Eingang des zentralen Kanals ein Sammelraum vorgesehen. Dieser Sammelraum erleichtert den Übergang von Schall und Luft aus dem Belüftungs- und Schallkanal in den zentralen Kanal. Dies ist vor allem dann von Vorteil, wenn mehrere Belüftungs- und Schallkanäle und/oder mehrere Nuten im zentralen Kanal beziehungsweise dem Zapfen vorgesehen sind.

Vorzugsweise ist, wie an sich bekannt, der Ventilkörper an der Stellschraube angeformt. Die Stellschraube sollte auch eine Verdrehhemmung aufweisen. Eine solche Verdrehhemmung kann aus einem im Gewinde der Stellschraube wirkenden elastischen Ring bestehen.

Obgleich es grundsätzlich möglich ist, den Belüftungsund Schallkanal im Gehäuse auszubilden, ist es aus fertigungstechnischen Gründen und zum Schutz vor Verschmutzung vorteilhafter, wenn, wie an sich bekannt, der Belüftungs- und Schallkanal in der Stellschraube ausgebildet ist.

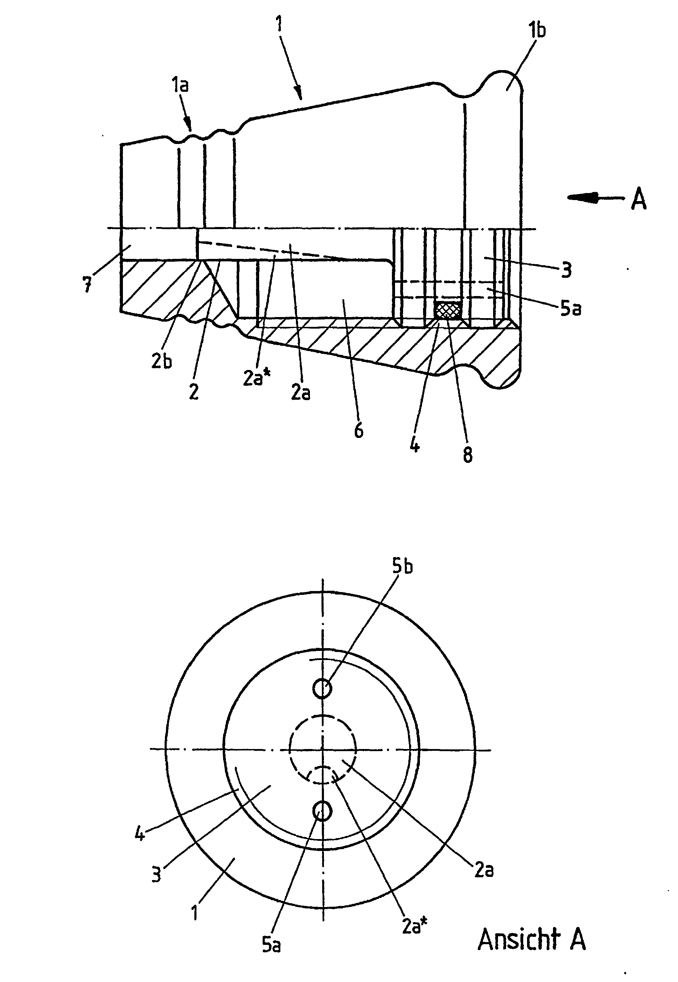

Im folgenden wird die Erfindung anhand einer ein Ausführungsbeispiel darstellenden Zeichnung näher erläutert. Die Zeichnung zeigt teils in Seitenansicht, teils im axialen Halbschnitt einen Gehörschutz sowie eine Vorderansicht des Gehörschutzes.

Der Gehörschutz besteht aus einem in der Zeichnung nicht dargestellten, dem Ohreingang des Trägers angepaßten, aus hautverträglichem, weichen Kunststoff bestehenden Formstück, auch "otoplastik" genannt und einem in einer entsprechenden Aufnahme eingesetzten Gehäuse 1 mit einem darin untergebrachten einstellbaren Ventil 2. Die individuelle Anpassung des Formstücks an den Ohreingang des Trägers dient dazu, um den Gehörschutz bei hohem Tragekomfort möglichst gut gegenüber dem Ohreingang abzudichten. Damit das Gehäuse 1 fest in der Aufnahme des Formstücks verankert werden kann, wird es eingeklebt. Zusätzlich sind im hinteren Bereich äußere Rillen 1a vorgesehen. Ein an der Stirnseite des Gehäuses 1 angeordneter Wulst 1b dient der leichteren Handhabung durch den Träger.

Das Ventil 2 besteht aus einem einstellbaren Ventilkörper 2a und einem Ventilsitz 2b. Der Ventilkörper 2a ist einstückig mit einer Stellschraube 3 ausgebildet, die in einem Gewinde 4 im Gehäuse 1 sitzt. Durch diese Stellschraube 3 erstrecken sich zwei von außen ausgehende Belüftungs- und Schallkanäle 5a,5b, die zu einem Sammelraum 6 im Gehäuse 1 führen, in dem der Ventilkörper 2a sich befindet. Von diesem Sammelraum 6 geht ein zur Innenseite führender zentraler Kanal 7 aus, der als zylindrische Bohrung ausgebildet ist. Der Eingang dieses zentralen Kanals 7 dient als Ventilsitz 2b für den als zylindrischer Zapfen ausgebildeten Ventilkörper 2a. Als Verdrehhemmung trägt die Stellschraube in einer Ringnut einen Gummiring 8.

Um den freien Durchgangsquerschnitt des Ventils 2 einzustellen, ist in dem Ventilkörper 2a eine Außennut 2a* vorgesehen, die in Richtung zur Innenseite einen zunehmenden Querschnitt aufweist. Dies ist im Ausführungsbeispiel dadurch verwirklicht, daß die Tiefe zur Innenseite zunimmt. Alternativ oder zusätzlich könnte die Außennut 2a* auch in der Breite zunehmen.

In der in der Zeichnung dargestellten Offenstellung ragt der Ventilkörper 2a mit seinem vorderen Ende geringfügig in den Eingang 2b des zentralen Kanals 7 hinein. Denkbar wäre aber auch, daß er mit seinem vorderen Ende geringfügig vor dem Eingang 2b des zentralen Kanals 7 liegt. In jedem Fall ist der maximale freie Querschnitt der Nut 2a* gegeben. Wird dann durch Drehen der Stellschraube 3, zum Beispiel mittels eines mit Stiften stirnseitig in die Belüftungs- und Schallkanäle 5a,5b eingreifenden Werkzeuges, der Ventilkörper 2 weiter in den zentralen Kanal 7 eingeführt, dann wird der wirksame freie Querschnitt der Nut am Eingang des zentralen Kanals 7 immer kleiner, bis die Nut 2a* vollständig im zentralen Kanal 7 liegt und der Durchgang verschlossen ist. Auf diese Art und Weise ist es möglich, feinfühlig den Durchgang für Luft und Schall einzustellen.

## Patentansprüche

1. Gehörschutz aus einem dem Ohreingang angepaßten Gehäuse (1) und einem darin untergebrachten Ventil (2), das zwischen mindestens einem von außen ausgehenden Belüftungs- und Schallkanal (5a,5b) und einem zur Innenseite führenden zentralen Kanal (7) angeordnet ist, dessen Eingang als Ventilsitz (2b) für dessen von außen über eine Stellschraube (3) einstellbaren Ventilkörper (2a) ausgebildet ist,
**dadurch gekennzeichnet, daß** der Ventilsitz (2b) als zylindrische Bohrung und der Ventilkörper (2a) als darin geführter, zylindrischer Zapfen ausgebildet sind, wobei zwischen der Bohrung (2b) und dem Zapfen (2a) mindestens eine sich in axialer Richtung erstreckende und in Richtung zur Innenseite im Querschnitt zunehmende Nut (2a*) vorgesehen ist, deren freier Durchgang von der Axialstellung des Zapfens (2a) zur Bohrung (2b) abhängt.

2. Gehörschutz nach Anspruch 1,
**dadurch gekennzeichnet, daß** die Nut (2a*) in dem Zapfen (2a) ausgebildet ist.

3. Gehörschutz nach einem der Ansprüche 1 oder 2,
**dadurch gekennzeichnet, daß** zwischen dem Ausgang des Belüftungs- und Schallkanals (5a,5b) und dem Eingang des zentralen Kanals (7) ein Sammelraum (6) vorgesehen ist.

4. Gehörschutz nach einem der Ansprüche 1 bis 3,
**dadurch gekennzeichnet , daß** der Ventilkörper (2) an der Stellschraube (3) angeformt ist.

5. Gehörschutz nach einem der Ansprüche 1 bis 4,
**dadurch gekennzeichnet , daß** die Stellschraube (3) eine Verdrehhemmung (8) aufweist.

6. Gehörschutz nach Anspruch 5,
**dadurch gekennzeichnet, daß** die Verdrehhemmung (8) aus einem zwischen den Gewinden der Stellschraube (3) und des Gehäuses (1) wirkenden elastischen Ring besteht.

7. Gehörschutz nach einem der Ansprüche 1 bis 6,
**dadurch gekennzeichnet, daß** der Belüftungs- und Schallkanal (5a,5b) in der Stellschraube (3) ausgebildet ist.

## Claims

1. Hearing protection comprising a casing (1) matched to the ear entrance and a valve (2) accommodated therein, which is arranged between at least one ventilation and sound channel (5a, 5b) running from outside and a central channel (7) running towards the inside, whose entrance is arranged as a valve seat (2b) for its valve body (2a) adjustable from outside by means of an adjusting screw (3),
**characterised in that**
the valve seat (2b) is constructed as a cylindrical hole and the valve body (2a) is constructed as a cylindrical pin guided therein, wherein between the hole (2b) and the pin (2a) there is provided at least one groove (2a*) which extends in the axial direction and increases in cross-section in the direction of the interior, whose free passage depends on the axial position of the pin (2a) with respect to the hole (2b).

2. The hearing protection according to claim 1,
**characterised in that**
the groove (2a*) is constructed in the pin (2a).

3. The hearing protection according to any one of claims 1 or 2,
**characterised in that**
between the outlet of the ventilation and sound channel (5a, 5b) and the inlet of the central channel (7) there is provided a collecting space (6) .

4. The hearing protection according to any one of claims 1 to 3,
**characterised in that**
the valve body (2) is moulded onto the adjusting screw (3).

5. The hearing protection according to any one of claims 1 to 4,
**characterised in that**
the adjusting screw (3) has a twist restraint (8).

6. The hearing protection according to claim 5,
**characterised in that**
the twist restraint (8) consists of an elastic ring acting between the threads of the adjusting screw (3) and the casing (1).

7. The hearing protection according to any one of claims 1 to 6,
**characterised in that**
the ventilation and sound channel (5a, 5b) is constructed in the adjusting screw (3).

## Revendications

1. Protection auditive constituée d'un boîtier (1) adapté au canal d'entrée de l'oreille et d'une valve (2) qui y est implantée, disposée entre au moins un canal aérateur et acoustique (5a, 5b) partant de l'extérieur et un canal central (7) conduisant vers la face interne, dont l'entrée est formée comme siège de valve (2b) pour le corps de valve (2a) de celle-ci, réglable de l'extérieur grâce à une vis de réglage (3),
**caractérisée en ce que** le siège de valve (2b) est formé d'un perçage cylindrique et le corps de valve (2a) d'un tenon cylindrique qui y est introduit, moyennant quoi entre le perçage (2b) et le tenon (2a) au moins une striure (2a*) à pas croissant transversal est prévue, s'avançant en direction axiale et en direction de la face interne, et dont la libre traversée dépend de la position axiale du tenon (2a) par rapport au perçage (2b).

2. Protection auditive selon la revendication 1,
**caractérisée en ce que** la striure (2a*) est formée dans le tenon (2a).

3. Protection auditive selon l'une des revendications 1 ou 2,
**caractérisée en ce qu'**entre la sortie du canal aérateur et acoustique (5a, 5b) et l'entrée du canal central (7) un espace collecteur (6) est prévu.

4. Protection auditive selon l'une des revendications 1 à 3,
**caractérisée en ce que** le corps de valve (2) est formé à proximité de la vis de réglage (3).

5. Protection auditive selon l'une des revendications 1 à 4,
**caractérisée en ce que** la vis de réglage (3) présente un blocage anti-torsion (8).

6. Protection auditive selon la revendication 5, **caractérisée en ce que** le blocage anti-torsion (8) est constitué d'un anneau élastique agissant entre les filetages de la vis de réglage (3) et du boîtier (1).

7. Protection auditive selon l'une des revendications 1 à 6,
**caractérisée en ce que** le canal aérateur et acoustique (5a, 5b) est formé dans la vis de réglage (3).
